# EUROPEAN PATENT APPLICATION

(11) **EP 1 431 305 A1**
(43) Date of publication of application: **23.06.2004**
(21) Application number: 03257879.1
(22) Date of filing: 16.12.2003
(51) Int. Cl.: C07J 31/00

(54) **Thiocarboxylic acid organic salts and processes utilizing the same**

(30) Priority: 16.12.2002 IL 15346202
(71) Applicant: CHEMAGIS LTD., Tel Aviv 61090 (IL)
(72) Inventor: Arad, Oded, Rehovot 76303 (IL); Brand, Michael, Raanana 43252 (IL); Saeed, Shadi, Haifa 33266 (IL); Davidi, Guy, Even Yehuda 40500 (IL)
(74) Representative: Fairbairn, Angus Chisholm

(57) **Abstract**

The invention provides a thiocarboxylic acid organic salt of the formula: 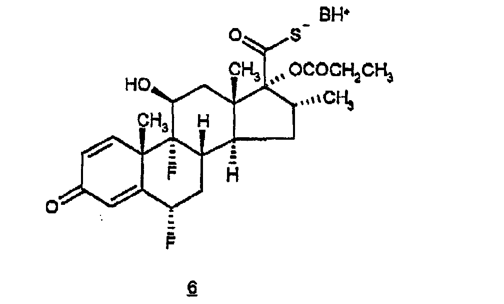 wherein B is an organic base.

## Description

The present invention relates to a method for the preparation and isolation of organic salts of 6α-9α-difluoro-11β-hydroxy-17α-propionyloxy-16α-methylpregna-3-oxo-1,4-diene-17β-thio-carboxylic acid in a pure state and to these compounds per se. These salts are useful for producing Fluticasone propionate of high purity.

6α-9α-difluoro-11β-hydroxy-17α-propionyloxy-16α-methyl-pregna-3-oxo-1,4-diene-17β-thiocarboxylic acid (1) is a key intermediate in the preparation of Fluticasone propionate (2), a well known antiallergic and anti-inflammatory corticosteriod that is a very efficient drug for the treatment of asthma, allergic and perennial rhinitis and topical inflammation.

Fluticasone propionate (2) is obtained from the thiocarboxylic acid (1) using chlorofluoromethane in the presence of a base as described in equation 1 according to the Israeli patent, IL 109656. 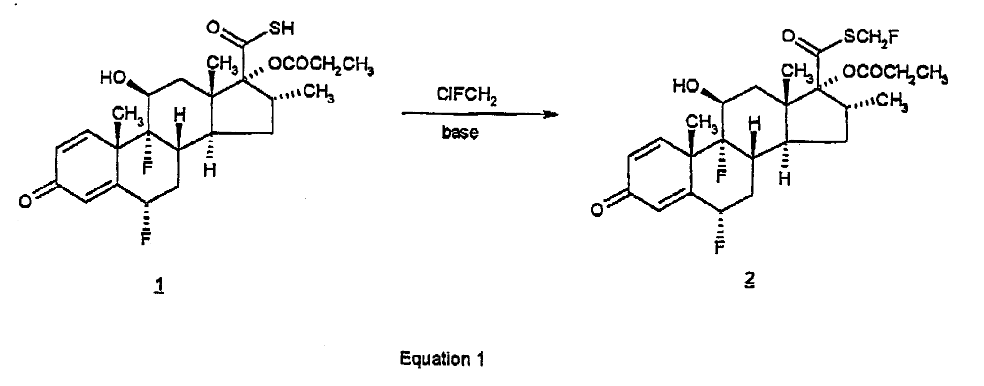 In J. Med. Chem. 37 (1994), 3720 the authors describe a process in which the potassium salt of the thiocarboxylic acid (3) is converted to the fluoromethyl thioester (2) using fluoroiodomethane or bromofluoromethane as described in equation 2. No experimental details are given as to how to obtain the potassium salt 3.

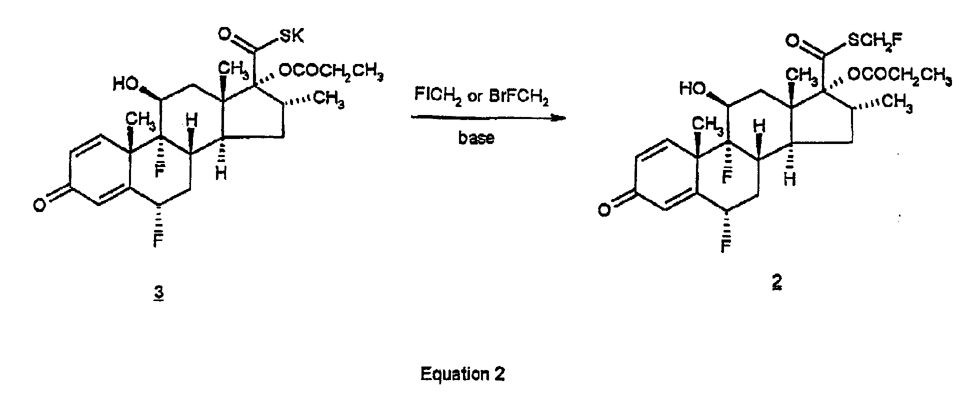

In a recent patent application, WO 0162722, the authors claim that compound 4 wherein M is Li, Na, or K. Compound 4 can be reacted with chlorofluoromethane in the presence of a second base such as NaHCO₃ to afford compound 2 as outlined in equation 3. The inorganic salt of 4 is not isolated but reacted in situ with chlorofluoromethane to afford compound 2.

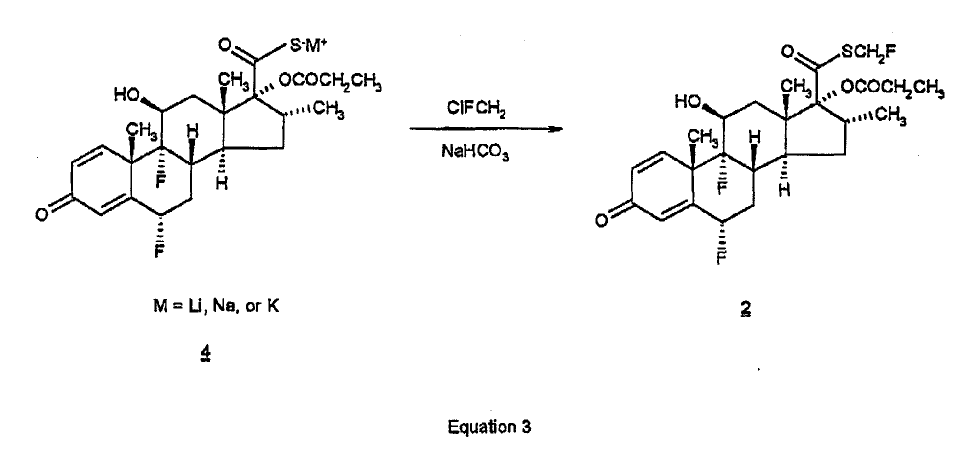

### SUMMARY OF THE INVENTION

The present invention relates to a process for isolating organic salts of 6α-9α-difluoro-11β-hydroxy-17α-(propionyloxy)-16α- methylpregna-3-oxo-1,4-diene-17β-thiocarboxylic acid (6) obtained as a result of the reaction of the thiocarboxylic acid (1) with an organic base B (5) in an organic solvent at temperatures in the range of 25°-60°C.

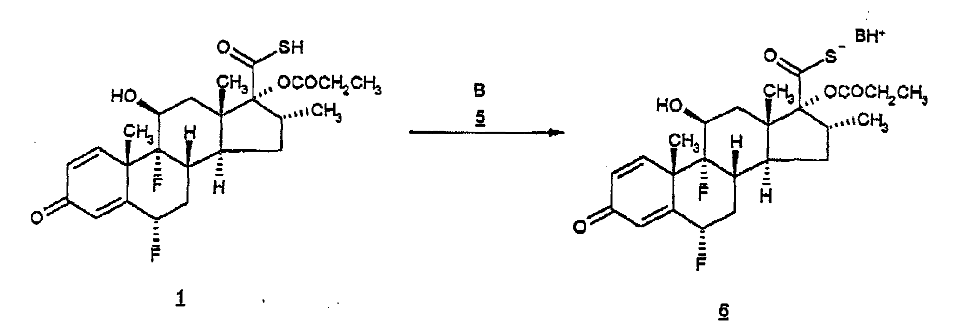

Thus according to the present invention there is now provided a thiocarboxylic acid organic salt of the formula: 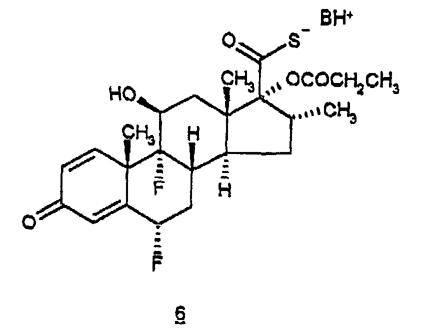 wherein B is an organic base.

In a preferred embodiment of this invention, B represents an organic amine of the general formula: 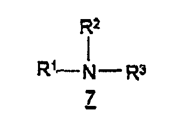 wherein the substituents R¹, R² and R³ are independently hydrogen, C₁- C₆ straight or branched alkyl, cycloalkyl, aryl, aralkyl, alkylene and alkyleneoxy.

In a preferred embodiment of this invention, the thiocarboxylic acid (1) is reacted with the organic amine (7) to afford the organic amine salt of 6α-9α-difluoro-11β-hydroxy-17α-(propionyloxy)-16α- methylpregna-3-oxo-1,4-diene-17β-thiocarboxylic acid (8). The preferred solvent for performing the reaction is isopropanol. The preferred temperature is at ambient temperature. The isolated thiocarboxylic acid organic amine salt (8) is reacted with chlorofluoromethane in acetonitrile at 50°C in a closed vessel to afford a highly pure form of Fluticasone propionate (2). The above sequence of reactions is outlined in the following scheme.

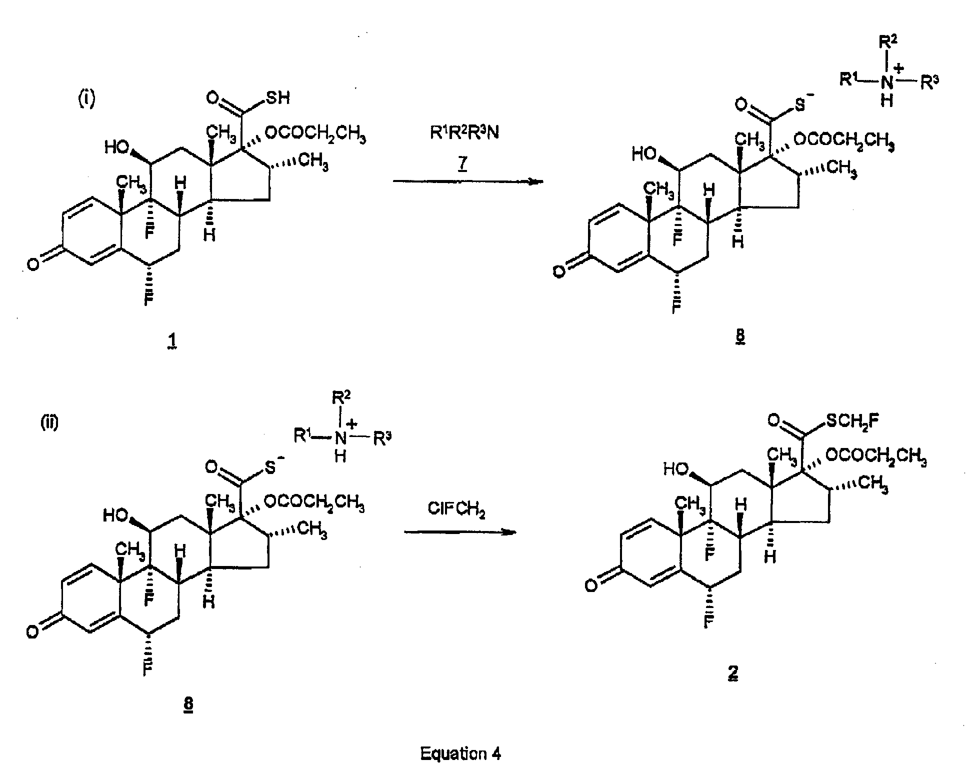

The substituents R¹, R² and R³ in formulae 7 and 8 are independently hydrogen, C₁ - C₆ straight or branched alkyl, cycloalkyl, aryl, aralkyl, alkylene and alkyleneoxy. The preferred organic amine of formula 7 is diisopropylethylamine.

According to the present invention, a process is now available for producing Fluticasone propionate of improved quality using an organic amine salt of 1.

The preparation of the organic amine salt 8 comprises the following steps:
(i) preparing a suspension of 1 in isopropanol at 25°-30°C
(ii) adding the organic amine (7) to the above suspension at 25°-30°C
(iii) stirring the resulting mixture at 25°-30°C for a specified period of time
(iv) cooling the reaction mixture to a temperature lower than 10°C
(v) separating the precipitated crystals by filtration

Surprisingly, it was found that the quality of Fluticasone propionate (2) was improved by reacting the isolated organic amine salts 8 with chlorofluoromethane. The improved quality of the organic amine salts 8 is evident by the HPLC chromatographic purity. In one instance, the purity of the thiocarboxylic acid 1 was increased from 91.7% to 94.8% for the isolated organic amine salt 8. The improvement in quality of the organic amine salt of 1 resulted in a purer Fluticasone propionate (2) prepared from the organic amine salt. In the majority of cases checked, the HPLC chromatographic purity of Fluticasone propionate in which the organic amine salt was not isolated was about 98% (see reference example). On the other hand, Fluticasone propionate prepared from isolated organic amine salt had an HPLC purity that was not less than 99.7%. Some of the major impurities that are reduced by this method are the thiocarboxylic acid acetate 9, the des propionyl thiocarboxylic acid 10, the carboxylic acid propionate 11 and disulphide 12.

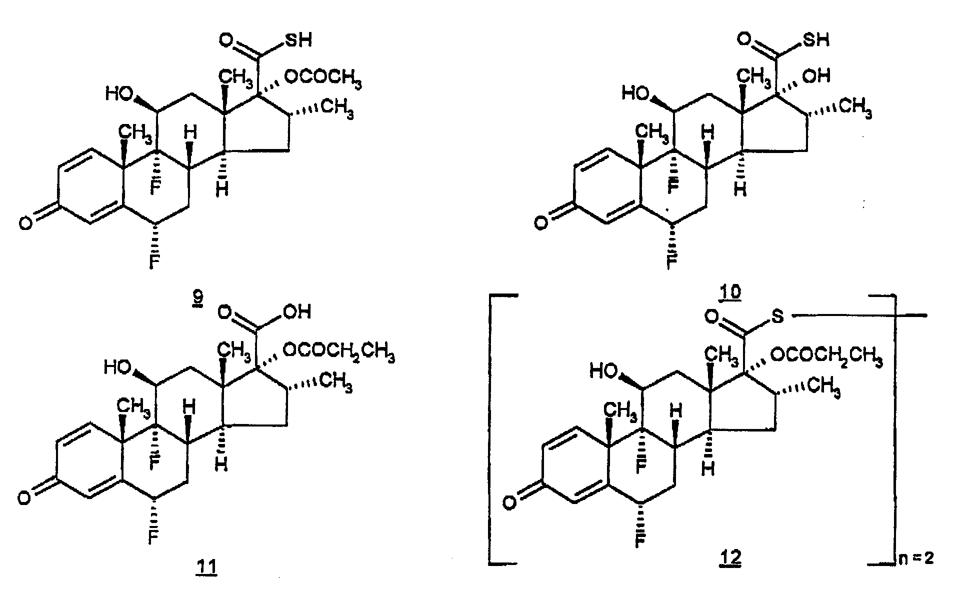

In addition, an attempt to purify 1 by crystallization proved to be inefficient.

By preparing and isolating the amine salt, some of the impurities will remain in solution and not precipitate with the amine salt. If the procedure of initially preparing the amine salt is not applied, some of the impurities will remain in the Fluticasone propionate.

The organic amine salts 8 are reacted with chlorofluoromethane in the following manner:
(i) preparing a mixture of the thiocarboxylic acid organic amine salt (8) in an appropriate solvent, preferably acetonitrile.
(ii) adding to this mixture about a two-fold excess of chlorofluoromethane
(iii) heating the mixture at 45°-50°C for a specified period of time
(iv) cooling the reaction mixture to a temperature lower than 10°C
(v) separating the precipitated crystals by filtration

It should be emphasized that although Fluticasone propionate can be prepared by reacting the thiocarboxylic acid (1) with chlorofluoromethane and an organic amine in acetonitrile, a purer product is obtained by using the isolated organic amine salt 8.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### EXAMPLES

### Example 1

### Step 1: Preparation of the Diisopropylethylamine salt of 1

The reaction vessel is charged with 10 g of the thiocarboxylic acid of 91.7% chromatographic purity (0.0214 moles, 1 eq.) and 30 ml isopropanol. To the stirred suspension is added 5.6 ml diisopropylethylamine (0.032 moles, 1.5 eq.) in portions at 25°C. The reaction is slightly exothermic. On completion of the addition a solution is obtained. The solution is stirred for 15 min. at 25°C after which time a suspension is formed. The mixture is cooled to 5°C and stirred at this temperature for three hours. The product is filtered, washed with cold isopropanol and dried at 50°C to afford 10.4 g of product in 82% yield. The HPLC chromatographic purity is 95.8%.

The product is characterized as follows:
m.p.: 182.7°C

### Step 2: Preparation of Fluticasone Propionate

A 200 ml glass autoclave is charged with 10 g of the diisopropylethylamine salt of the thiocarboxylic acid (0.0167 moles, 1 eq.) and 50 ml acetonitrile. Into the stirred suspension is bubbled 2.55 g chlorofluoromethane (0.037 moles, 2.2 eq.). The autoclave is closed. The stirred suspension is heated to 50°C to afford a solution after about 30 min. A pressure of about 1.3 bar is produced. The mixture is heated at 50°C for a period of 5 hours during which time a suspension is produced. After the heating period, the vent is opened and the mixture cooled to 25°C. The mixture is stirred at 25°C for 16 hours (overnight). The suspension is cooled to 5°C and stirred at this temperature for 1 hour. The product is filtered, washed with cold acetonitrile and dried at 60°C to afford 5.0 g of product in 60% yield. The HPLC chromatographic purity is 99.86%.

### Examples 2 - 5

In a similar fashion, a series of experiments were performed using the following organic amines: triethylamine, 1-methylpiperidine, diethylamine, morpholine, dicyclohexylamine and cyclohexylamine. For triethylamine, 1-methylpiperidine, diethylamine and morpholine the resulting isolated organic amine salts were converted to Fluticasone propionate as described above. In the case of dicyclohexylamine and cyclohexylamine the organic amine salts were not obtained in crystalline form but rather as an oil which was reacted with chlorofluoromethane to afford Fluticasone propionate. The results are listed in the following table.

| Example | Organic Amine | HPLC Purity (%) | | |
|---|---|---|---|---|
| | | Thiocarboxylic acid | Thioacid salt (mp) | Fluticasone propionate |
| 2 | Triethylamine | 89.4 | 93.9 (161.7°C) | 99.7 |
| 3 | 1-methyl-piperidine | 90.4 | 96.2 (182.1°C) | 99.7 |
| 4 | Diethylamine | 88.8 | 95.0 (156.4°C) | 99.7 |
| 5 | Morpholine | 89.1 | 95.6 (162.7°C) | 81.6 |
| 6 | Dicyclohexylamine | 88.8 | 87.5 (oil) | 98.9 |
| 7 | Cyclohexylamine | 88.8 | 86.3 (oil) | 90.5 |

On the basis of the results presented in Example 1 and Examples 2 - 5, the tertiary thioacid amine salts ― diisopropylethylamine, triethylamine and 1-methylpiperidine ― that were studied were the preferred ones for producing Fluticasone propionate (2) of high quality.

The following example is provided to illustrate the results obtained when the thiocarboxylic acid 1 is reacted directly with chlorofluoromethane to afford Fluticasone propionate.

### Reference Example for the Preparation of Fluticasone Propionate

### Preparation of Fluticasone Propionate

A 200 ml glass autoclave is charged with 7.8 g of the thiocarboxylic acid (1) of 91.7% chromatographic purity (0.0167 moles, 1 eq.) and 50 ml acetonitrile. To the stirred suspension is added 4.4 ml diisopropylethylamine (0.026 moles, 1.5 eq.). The mixture is stirred for 15 min. to afford a clear yellow solution. Into the stirred solution is bubbled 2.55 g chlorofluoromethane (0.037 moles, 2.2 eq.). The autoclave is closed. The stirred suspension is heated to 50°C to afford a solution after about 30 min. A pressure of about 1.3 bar is produced. The mixture is heated at 50°C for a period of 5 hours during which time a suspension is produced. After the heating period, the vent is opened and the mixture cooled to 25°C. The mixture is stirred at 25°C for 16 hours (overnight). The suspension is cooled to 5°C and stirred at this temperature for 1 hour. The product is filtered, washed with cold acetonitrile and dried at 60°C to afford 5.0 g of product in 60% yield. The HPLC chromatographic purity is 98%.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A thiocarboxylic acid organic salt of the formula: 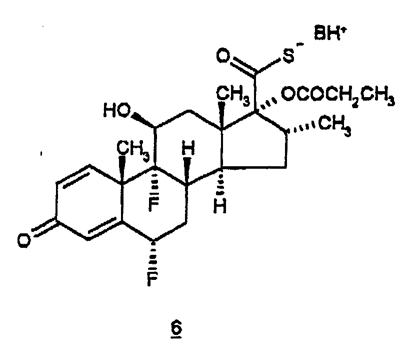 wherein B is an organic base.

2. A thiocarboxylic acid organic salt according to claim 1 where B is an organic amine of the general formula: 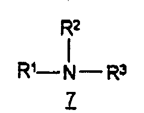 wherein R¹, R² and R³ are independently hydrogen, C₁ - C₆ straight or branched alkyl, cycloalkyl, aryl, aralkyl, alkylene and alkyleneoxy.

3. A thiocarboxylic acid organic salt according to claim 2 wherein said organic amines B are selected from the group consisting of diisopropylethylamine, triethylamine and 1-methylpiperidine.

4. A process for preparing highly pure Fluticasone propionate which comprises reacting a thiocarboxylic acid organic salt of formula 6 as defined in claim 1 and chlorofluoromethane.

5. A process according to claim 4 wherein the thiocarboxylic acid salt 8 is prepared by reacting 1 with an organic amine of formula 7 comprising the following steps: 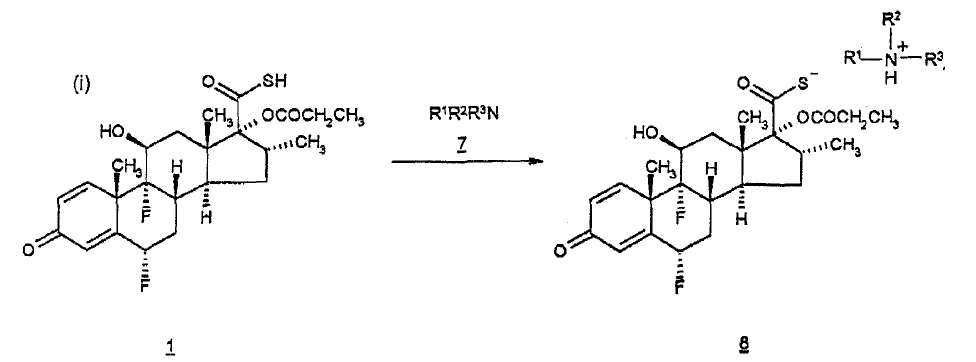
(i) preparing a suspension of 1 in an appropriate solvent;
(ii) adding the organic amine (7);
(iii) stirring the mixture at a temperature range 1;
(iv) cooling the mixture to a temperature range 2; and
(v) separating the crystals that are formed.

6. A process according to claim 5 wherein the solvent is isopropanol.

7. A process according to claim 5 wherein the temperature range 1 is 25°-60°C.

8. A process according to claim 5 wherein the temperature range 2 is -10°-10°C.

9. A process according to claim 5 wherein the separation of crystals is done by filtration.

10. A process according to claim 4 wherein the Fluticasone propionate is . produced according to the following steps:
(i) preparing a mixture of the isolated thiocarboxylic acid organic amine salt (8) 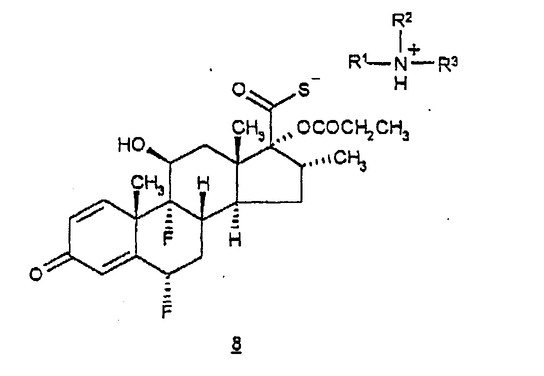 in acetonitrile;
(ii) adding to this mixture about a two-fold molar excess of chlorofluoromethane;
(iii) heating the mixture at 50°C for a specified period of time;
(iv) cooling the reaction mixture to a temperature lower than 10°C; and
(v) separating the precipitated crystals by filtration.

11. A process according to claim 5 wherein the thiocarboxylic acid organic amine salts are selected from the group consisting of
6α,9α-difluoro-11β-hydroxy-7α-propionyloxy-16α-methylpregna-3-oxo-1,4-diene-17β-thiocarboxylic acid diisopropylethylamine salt, triethylamine salt and N-methylpiperidine salt.

12. Fluticasone propionate prepared as described in claim 10.
